(19) Europäisches Patentamt
European Patent Office
Office européen des brevets

(11) Publication number: **0 269 449 A2**

(12) **EUROPEAN PATENT APPLICATION**

(21) Application number: **87310465.7**

(22) Date of filing: **26.11.87**

(51) Int. Cl.$^4$: **C 08 J 9/00**
C 08 L 27/00, A 61 L 27/00

(30) Priority: **26.11.86 US 935237**

(43) Date of publication of application:
**01.06.88 Bulletin 88/22**

(84) Designated Contracting States: **DE FR GB IT**

(71) Applicant: **BAXTER TRAVENOL LABORATORIES, INC.**
**One Baxter Parkway**
**Deerfield, IL 60015 (US)**

(72) Inventor: **Tu, Roger**
**24755 Scott Lane**
**Lake Forest City California 92630 (US)**

**Wang, Edwin**
**10 Varessa**
**Irvine California 92720 (US)**

(74) Representative: **Tubby, David George et al**
**MARKS & CLERK 57-60 Lincoln's Inn Fields**
**London WC2A 3LS (GB)**

(54) **Porous flexible radially expanded fluoropolymers and process for producing the same.**

(57) An interpenetrated matrix of a polytetrafluoroethylene and an elastomer is formed into a composite material which is flexible, durable and highly porous with a tight microporous structure and has a high rate of retractability. The composite material is subjected to radial expansion to produce shaped articles such as tubes and rods with excellent compliance, flexibility and elasticity. More particularly, the composite material can be utilized to produce vascular grafts with excellent biological compatibility that are capable of dynamic local diameter change responsive to pulsatile blood pressure change.

EP 0 269 449 A2

## Description

## POROUS FLEXIBLE RADIALLY EXPANDED FLUOROPOLYMERS AND PROCESS FOR PRODUCING THE SAME

The present invention relates to a method of producing flexible articles of poly(tetrafluoroethylene) which are especially useful as medical grafts and implants.

Co-pending European application No. 87306887.8 discloses the use of elastomers which strengthen expanded poly(tetrafluoroethylene) fibrils by forming a continuous matrix interpenetrating the microstructure of the fibrils. In so doing, they render the poly(tetrafluoroethylene) structure porous but yet durable with excellent pliability for use as a vascular graft. More importantly, however, addition of an elastomer to the poly(tetrafluoroethylene) allows a vascular graft made from the material to be biologically compatible with surrounding tissue.

While the process disclosed in EP Application 87306887.8 can be used to formulate a useful poly(tetrafluoroethylene) product, this invention relates to an additional process and improved product where there is an improvement in the compliance, elasticity, flexibility and strength of the poly(tetrafluoroethylene)-elastomer matrix due to novel radial expansion steps in the process. This invention can be applied to rods and tubes, or any products produced thereby, but more particularly relates to medical implants as shaped articles produced from porous poly(tetrafluoroethylene). In this invention, the emphasis is directed to a process for producing, and the improved physical characteristics of, medical implants or specifically, vascular grafts and shaped articles of poly(tetrafluoroethylene), and is not intended to limit the application of the process and other products described herein.

Thus, the present invention consists in a process for the production of a porous compliant shaped article from a poly(tetrafluoroethylene) polymer, which process comprises first expanding a shaped article comprising poly(tetrafluoroethylene) crossed-linked with at least one elastomer, heating the unsintered shaped article at a temperature and for a time sufficient to cross-link said at least one elastomer to poly(tetrafluoroethylene), sintering the shaped article and radially expanding the sintered article.

To manufacture a highly porous poly(tetrafluoroethylene) material, it is generally difficult to radially expand the material for fear of cracking and breaking. The radial expansion process of the present invention yields a compliant material with essentially all the fibrils intact. The poly(tetrafluoroethylene)elastomer matrix is preferred because the pre-mixed, interpenetrated elastomer tends to absorb the stretching energy in the radial expansion steps of the process.

US 2 586 357 and 3 953 566 and Japanese Patent Publication 13560/1967 describe conventional wet paste formulations and processes for producing poly(tetrafluoroethylene) products.

Conventional vascular grafts manufactured from porous poly(tetrafluoroethylene) have limitations in their strength and compliance. The porous grafts do not hold or resist dilation unless wrapped with a reinforcing film for support. This reinforcement slows down the tissue ingrowth, preventing rapid healing. This is because of the relatively low radial tensile strength of poly(tetrafluoroethylene). In addition, the grafts are stiff and non-compliant to the natural artery. A porous flexible structure that is closer in compliance to the natural vessel will help prevent the complications resulting from the aforementioned detrimental characteristics.

Shaped articles, including medical implants, can be produced from poly(tetrafluoroethylene) and an elastomer. Examples of such elastomers include fluorinated ethylene propylene, polyvinylidene fluoride co-hexafluoropropylene, poly(tetrafluoroethylene coperfluoromethylvinylether), poly(tetrafluoroethylene-co-propylene), poly(vinylidene-co-chlorotrifluoroethylene), silicones, fluorosilicones, fluoroalkoxy phosphazenes, segmented copolyester ether, styrene butadiene block copolymers, polyethers, acrylonitrile butadienes, isoprenes and mixtures of any two or more thereof. The process for producing said shaped articles involves radial expansion of the poly(tetrafluoroethylene)elastomer-matrix.

The shaped articles of poly(tetrafluoroethylene) and an elastomer produced in accordance with this invention have improved compliance, flexibility, pliability, elasticity and biological compatibility.

The invention is further illustrated by the accompanying drawings, in which:

FIGURES 1 and 2 are schematic representations of the process of this invention.

FIGURE 3 depicts a rod utilized for radially enlarging a tube.

FIGURE 4 depicts the tip of a tube being fitted on the neck and shoulders of the rod of Figure 3.

FIGURE 5 depicts a tube being radially enlarged by the rod of Figure 3.

FIGURES 6 through 9 are photomicrographs of products of this invention as shown in the Examples.

Porous poly(tetrafluoroethylene) vascular grafts are fabricated by the process described in EP Application 87306887.8 by paste extrusion followed by the novel heating and stretching of the extruded polymer composite in accordance with the present invention.

It has been found that composite materials made generally in accordance with the teachings of EP Application 87306887.8 that are paste formed, dried unsintered shapes which are expanded by radially stretching under certain conditions are suitable for the manufacture of improved medical implants that have compliance similar to a natural artery. Compliance is generally defined as the fractional change in luminal volume that occurs in response to a unit change in pressure. The process includes the addition of one or more elastomers to the poly(tetrafluoroethylene) resin prior to forming. The elastomer is preferably a polyvinylidene fluoride-co-hexafluoroepropylene, poly[tetrafluoroethylene-co-perfluoromethylvinylether], poly(tetrafluorethylene-co-propylene), poly(vinylidene-co-chlorotrifluoroethylene), silicone, fluorosilicone, fluoroalkoxy phosphazene, segmented copolyester ether, styrene butadiene block copolymer, polyether, acrylonitrile butadiene, isoprene or mixture thereof. The elastomer may be added to the poly(tetrafluoroethylene) in amounts effective to produce the desired results in a medical implant. Preferably, these amounts range from about 2% by weight to about 50% by weight of the shaped article. More preferably, the amount of elastomer that should be added to the poly(tetrafluoroethylene) to produce the desired results of this invention is about 10% by weight of the poly(tetrafluoroethylene). Preferably, it is desired to add more than one elastomer to the poly(tetrafluoroethylene), but not more than two. However, the total weight should not exceed the aforementioned 2% to about 50% by weight range.

While any of the aforementioned elastomers function in this invention, a co-polymer of propylene and tetrafluoroethylene, i.e. poly(tetrafluoroethylene-co-propylene), sold under the tradename Aflas, manufactured by Asahi Glass Company, is preferred. This has a structure in which tetrafluoroethylene and propylene arrange alternately in an orderly manner as shown:

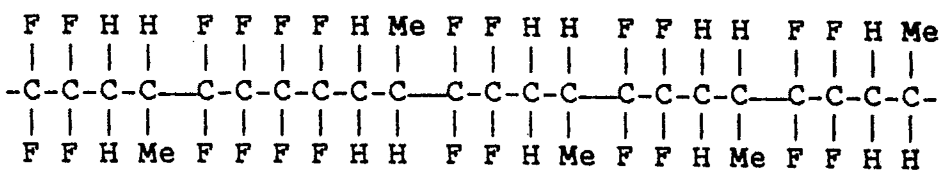

(Me = methyl).

The tight microporous structure obtained by the process of this invention is composed of finely oriented fibres of poly(tetrafluoroethylene) coated with the elastomer. The elastomer encapsulates and reinforces the poly(tetrafluoroethylene) fibrils which interconnect nodes. The elastomer is a continuous matrix interpenetrating the microstructure of the fibrils. It modifies the sharp edges of the fibrils and nodes to render the edges smooth. The smooth edges of the fibrils and nodes create a poly(tetrafluoroethylene) elastomer product resistant to suture tearing with high intrinsic elasticity. In the case of radial expansion, the nodes are elongated perpendicular to the axial direction. The fibrils appear to be characteristically wide in cross-section, the maximum width being equal to about 5 microns. The minimum width may be about 0.1 to about 0.5 microns. The nodes may vary in size from about 5 microns to about 50 microns, depending on the conditions used in the expansion, and the amount and type of elastomer added to the poly(tetrafluoroethylene). Shaped articles have been expanded at high temperatures and rates that exhibit a tight microporous, homogeneous structure that have closely spaced nodes interconnected with a great number of fibrils. The structure preferably has a specific gravity ranging from about 0.2 to about 1.3, more preferably about 0.6 and a porosity ranging from about 40% to about 90% void. The porosity allows for tissue ingrowth without allowing red blood cells to pass through the walls of the graft. The porous structure is opaque, however permeable to gases. The grafts are biologically compatible in that they function within normal biological actions and are not rejected. The elastomer present in the graft absorbs the kinetic energy of pulsating blood, thus providing durability to the graft that is missing in conventional grafts made from poly(tetrafluoroethylene) alone. The grafts are compliant in that they distend and retract radially as a function of the pulsatile blood pressure.

In accordance with the present invention, the inside diameter of a tube or graft may be increased, preferably up to about 100% of its original diameter while retaining a tight microporous structure with adequate porosity to promote biological compatibility. More preferably, the inside diameter of a tube or graft is enlarged to about 10% to about 50% of its original diameter.

The matrix tensile strength of the porous structure of this invention is normally in the range of 6000-7000 psi. In Example 7 which follows, a method for calculating matrix tensile strength appears. The values achieved appear to be relatively high, and the structure is strong but pliable and elastic. The structure, unlike prior art devices, is not stiff and possesses the characteristic compliance, pliability, durability and biological compatibility needed to enable it to function as a medical implant.

As stated, the process of EP Application 87306887.8 can be utilized to produce the porous expanded paste-formed products of this invention. Typically, processes such as described in US 3 953 566 may also be utilized in conjunction with the addition of an elastomer described herein to produce a composite material that can be formed into shaped articles having excellent pliability, elasticity and durability. Any conventional process for obtaining an unsintered moulding by a paste extrusion process, the calendering process or a combination of them may be utilized with the addition of an elastomer to produce the unexpected results described herein.

Accordingly, the polymeric matrix of this invention may be obtained by combining dispersions or emulsions of poly(tetrafluoroethylene) and the elastomer and coagulating the blend. Dispersions of poly(tetrafluoroethylene) will normally contain about 10 to about 50% poly(tetrafluoroethylene) in water. More preferably, about 25% by weight is utilized. The elastomer dispersion preferably contains about 10 to 50% by weight of the elastomer. More preferably, about 25% by weight is utilized to provide a mixture with the

poly(tetrafluoroethylene) in the blended dispersions. The amounts of each of the poly(tetrafluoroethylene) and elastomer dispersions to form the blended dispersion may be varied so that the elastomer may be present in the polymeric matrix in amounts ranging from about 2% by weight to about 50% by weight (of the shaped article).

After coagulation, e.g. with acetone, the coagulate may be filtered, washed with ethanol and dried. At this point it may be mixed with a lubricant. Alternatively, at this point, the process of producing the polymeric matrix may begin if one desires to use poly(tetrafluoroethylene) powder. Poly(tetrafluoroethylene) in dry powder form, which is commercially available, may be admixed with a dispersion or emulsion of an elastomer. For example, the elastomer may be dispersed in solvent and sprayed on the poly(tetrafluoroethylene) powder while the powder is tumbling. The spraying should be accomplished on an intermittant basis to avoid lumping. Regardless of the source of the poly(tetrafluoroethylene), the blended poly(tetrafluoroethylene)elastomer-lubricant mixture is left to stand to allow for lubricant dispersion.

The powder is then moulded by extrusion, rolling or a combination thereof. For a paste extrusion process, the powder is compressed to form a preform or billet which is extruded under conditions of elevated temperature and pressure.

The cut extrudate is preferably heated to a temperature between 35°C to 342°C. First, expansion of the extrudate is accomplished uniaxially. With reference to uniaxial expansion, the nodes are elongated, the longer axis of a node being oriented perpendicular to the direction of expansion. The fibrils are oriented parallel to the direction of expansion. The rate of stretch may vary and is not intended to be limiting in each of the expansion steps. In the axial expansion step the preferred rate of stretch is 10% per second. Another preferred rate of stretch is 100% per second. In general, the preferred rate of stretch is anything from 10 to 100% per second. The extrudate may be stretched more than once. After the first stretching, the material is heated to about 175°C for about 30 minutes to cure the elastomer present in the poly(tetrafluoroethylene) matrix so cross-linking occurs and to sinter the poly(tetrafluoroethylene).

In general, we prefer that the elastomer is cross-linked at a temperature from 150°F (65°C) to 350°F (177°C), and preferably for a period of from 5 to 60 minutes.

The radial expansion step preferably requires stretching the tube over a rod as depicted in Figures 3 to 5. The expanded extrudate is retracted axially to shorten its diameter by reorienting the fibrils. Retraction occurs by shortening the length of the expanded extrudate on a mandril. About 5% to about 60% of the length of the extrudate can be retracted, more preferably 30%. The expanded extrudate is radially enlarged by placing on the rod depicted in Figures 3-5. Figure 3 shows rod A with a neck 1, shoulder 2, round body 3 and base 4. The diameter $D_2$ is the final diameter that a tube 5 can be enlarged to. The internal diameter of the tube $D_3$ is larger than neck diameter $D_1$ but smaller than body diameter $D_2$.

The tube 5 is placed over neck 1 and pulled over shoulder 2 onto round body 3 under temperatures preferably ranging from room temperature to about 250°F (121°C). After tube 5 is pulled onto round body 3 of rod A the desired distance, tube 5 may be heated at a temperature of about 300°F (148°C) to 600°F (316°C), preferably about 400°F for about 10 minutes to anneal the poly(tetrafluoroethylene) and elastomer present therein.

Ater expansion, the tube is removed from the rod and is sintered by insertion into an oven at temperatures preferably ranging from 342°C to 390°C for a short period of time.

With respect to an alternative preferred step, a sintered matrix poly(tetrafluoroethylene that has not been impregnated with an elastomer may be immersed in an elastomer dispersion containing from about 1 to about 10% by weight elastomer before radial expansion. This step helps to rejoin any unconnected fibrils in the matrix. The elastomer solvent is evaporated while the tube is retracted to a predetermined shorter length on a mandril. This is accomplished by evenly shortening the tube length on a mandril. After retraction, the tube is preferably again soaked in the elastomer dispersion for about 10 minutes while the tube is compacted or restrained radially by exterior pressure via tightened tape or deflated balloon. The tube can be dried while on the radial restraint to provide a compliant vascular graft. Conventionally produced poly(tetrafluoroethylene) products may absorb up to about 10% by weight elastomer from the elastomer dispersion. As a result, their physical properties are improved, i.e., pliability, elasticity, etc. and they become suitable for use as compliant medical implants.

As indicated, while the radially expanded graft is compliant, additional steps can be taken to improve its compliance. For a poly(tetrafluoroethylene)/elastomer graft to have increased compliance, the elastomer must function effectively between the diastolic diameter and systolic diameter, which respectively correspond to the diastolic and systolic blood pressures. By radially expanding a poly(tetrafluoroethylene)/elastomer graft as described in the present invention, the systolic diameter is created and set. Improved compliance will result when the diastolic diameter of the radially enlarged vascular graft is set by compaction or restraint. The poly(tetrafluoroethylene) in the vascular graft of the present invention functions like collagen in an artery where it is wrinkled or relaxed for it has a vessel diameter less than its systolic diameter. The vascular graft is very elastic when the graft diameter is less than its systolic diameter because the elastomer portion of the present invention functions like the elastin in an artery. Without compacting or restraining, while the elastomer is wet or being soaked in its solvent and then subsequently dried, the elastomer may not exert its influence within the range between the diastolic diameter and systolic diameter. Accordingly, for greatly improved compliance, the compacting or restraining step is required.

Compacting or restraining requires the reduction in size of the radially enlarged poly(tetrafluoroethylene)/elastomer graft so that it becomes the diameter substantially that of the diameter of the graft during diastolic pressure. Usually, this is about 75% to about 95% of the inside diameter of the radially enlarged graft. The graft can be compacted or restrained by any suitable means. For example, woven dacron tape or a latex balloon or even pressurized air can be used to compact the radially expanded vascular graft against a mandril. The graft may be wrapped around a porous mandril, the compacting means applied, with solvent being applied to the graft through the holes in the porous mandril. The solvent partially dissolves the elastomer present in the graft. After an effective amount of time. e.g. about 5 to about 30 minutes, the solvent is allowed to evaporate so the elastomer is dried and fixed at the compacted diameter, which corresponds to the diastolic diameter which occurs under diastolic pressure. The prior radial enlargement of the graft reduces substantially the resistance of the poly(tetrafluoroethylene) portion of the graft to distend from the diastolic diameter to the systolic diameter.

For conventionally produced non-retracted and non-radially expanded synthetic grafts made from poly(tetrafluoroethylene), the graft length is constant during a pulsatile systolic and diastolic pressure cycle. In most instances, the compliance is thereafter simplified as the fractional diameter change per unit pressure change. In accordance with the present invention, the radially expanded, retracted poly(tetrafluoroethylene)/elastomer graft is longitudinally elastic, for the dynamic local diameter change or luminal volume change in response to the pulsatile blood pressure change generally follows the blood pressure wave form which is a sinusoidal curve which transmits the wave form to the distal end of the graft.

As is evident from the foregoing, the elastomer may be added to the poly(tetrafluoroethylene) by any number of methods within the knowledge of those skilled in the art.

As illustrated in Figures 1 and 2, a typical process for producing an interpenetrated polymeric matrix of poly(tetrafluoroethylene) and an elastomer is described as follows:

Step 1--Blending: Aqueous dispersions of poly(tetrafluoroethylene) and a fluoroelastomer are blended with very mild stirring. About 25% by weight elastomer, such as Aflas, is dispersed in water. About 25% by weight poly(tetrafluoroethylene) is dispersed in water and the solutions are combined.

Step 2-- Coagulating: The blended dispersion is then coagulated by adding acetone while agitating vigorously. The coagulum floats to the top.

Step 3--Filtering: The coagulum is vacuum filtered to remove as much water and acetone as possible.

Step 4--Washing: The filtered coagulum is then washed repeatedly with ethanol and water to extract the surfactant which was in the suspension.

Step 5--Drying: After washing the coagulum, now a powder, it is dried to volatilize any entrapped water or ethanol.

Step 6--Blending/Compounding: The poly(tetrafluoroethylene) powder is combined with about 10 to 30% by weight of a hydrocarbon to serve as a lubricant. Examples of suitable hydrocarbons include kerosene. mineral spirits, alcohol, glycol, and aromatics. The blended powder must preferably stand several hours for uniform lubricant dispersion.

Step 7--Preforming: The lubricated powder is placed into a cylinder with a core rod in the center. The powder is then compressed to 300 to 500 psi, which forms a solid form called a preform or billet.

Step 8--Paste Extrusion: The preform is placed in an extruder which, under e.g. hydraulic pressure, forces the material out of the die. The extrudate material is then cut into sections of predetermined lengths.

Step 9--Expansion: The extrudates are dried to evaporate the lubricant. As a result of the addition of the elastomer, the expansion parameters, such as temperature, rate, and ratio, can be augmented with equally good results. Generally, the tubes are heated within a temperature range of about 35° C to about 342° C, preferably to about 300° C, which is below the crystalline melting point. The tubes are expanded one or more times within the foregoing temperature range. After the desired axial expansion at stretch rates preferably ranging from about 10% per second to 100% per second, the tubes are heated, preferably at about 175° C for about 30 minutes, to cure the elastomer. The graft may then be sintered, e.g. at temperatures ranging from about 342° C to about 390° C for a relatively short period of time.

Step 10--Retraction: The tube may preferably be retracted by conventional techniques to about 5 to about 60% of its stretched length.

Step 11--Radial exapansion: The tubes may be radially expanded as previously described.

Step 12--Compaction: Since the poly(tetrafluoroethylene) portion of the graft is set for the systolic pressure diameter in the radial expansion, compaction will set the elastomer portion of the graft for the diastolic pressure diameter of the graft. Compaction or restriction of the tube or graft may involve wrapping the graft around a porous mandril through which a solvent, such as a liquid fluorocarbon (e.g. Freon® TF) manufactured by Dupont, tetrahydrofuran (THF) or 1,1,1-trichlorethane flows to contact the tube to partially dissolve the elastomer. Porous woven tape suitably about 1 cm wide or latex can be applied spirally around the tube wrapped on the mandril to compact it. For example, a wrapping angle of 70° with respect to the axis of the mandril may be used for wrapping a graft with an outer diameter of 7 mm. Overlapping of the tape past the ends of the graft by a small distance is preferred. Optionally, pressurized air may be blown on the graft to compact it. The solvent soaks the graft for about 30 minutes and then the flow is stopped to allow the solvent to evaporate. After drying of the graft, the tape or latex is unwrapped and the compliant graft is removed from the mandril. The graft is in its pre-radially expanded shape. The elastomer is set for the diastolic pressure diameter of the graft.

Step 13--Trim and Package: The graft may then be trimmed and packaged for distribution.

The addition of elastomers to poly(tetrafluoroethylene) and radial expansion improve the physical properties of medical implants and, specifically, vascular grafts made therefrom. Among these properties are compliance, handling and suture retention, elasticity, pliability, durability, and biological compatibility.

The following examples describe the processes and products within this invention as well as a further description of the properties of the expanded poly(tetrafluoroethylene) polymers/elastomers. As indicated above, some of the properties of these expanded products are substantially different from the corresponding properties of conventional extruded or moulded tetrafluoroethylene polymers. As a result of these differences, the radially expanded composite material is useful in many applications involving medical implants and vascular grafts.

EXAMPLE 1

JSR Aflas elastomer, a copolymer of propylene and tetrafluoroethylene manufactured by Asahi Glass Company, was dissolved in ethyl acetate and diluted with Freon TF, manufactured by Dupont, in about a 10% by weight solution. The diluted elastomer solution was then mixed with poly(tetrafluoroethylene) powder while tumbling. The poly(tetrafluoroethylene) powder is sold under the trade name Fluon CD123 and manufactured by ICI Americas.

About 20 percent mineral spirits lubricant, on the final solids basis, was added to the poly(tetrafluoroethylene)-elastomer mixture. The mixture was exposed to air for solvent evaporation. The final lubricant concentration was controlled at about 12% by weight. The lubricated powder was placed into a cylinder with a core rod in the center. The powder was then compressed to 300 to 500 psi, which formed a solid form called a preform or billet. The preforms were cut into 4 inch (10 cm) lengths with 6 mm inside diameter. They were loaded into an expansion oven. The temperature in the oven was raised to about 400°F (204°C) while the preforms were expanded at an expansion rate of about 10% per second. The expanded preforms were removed from the oven and loaded on a separate rack for sintering. The empty oven was heated to 700°F (371°C). The rack holding the expanded preforms was loaded into the oven for flash sintering of the preforms. The sintered preforms were used in the following examples.

EXAMPLE 2

Sintered tubes from example 1 were loaded on a 6 mm mandril at 20% retraction. Both ends of each tube were tied onto the mandril. The tubes were then soaked in a solution containing Aflas at a level of 2%, 5% and 10% by weight of Freon TF manufactured by DuPont and dried thereafter. The soaked tubes were coded Samples 1A, 1B, and 1C, respectively. They all showed good longitudinal elasticity. The control poly(tetrafluoroethylene) vascular grafts made conventionally by Impra were coded Samples 1D and 1E, respectively.

Samples 1B, 1C and 1D were radially enlarged from 6 mm to 7 or 8 mm internal diameter on the rod described in Figures 3 to 5. The enlarged tubes were, thereafter, coded 2B, 2C and 2D, respectively.

EXAMPLE 3

Sample 2B was loaded onto a 6 mm mandril, and tied at both ends. A woven Dacron tape was wrapped with some tension around the sample to force the radially enlarged sample tightly against the mandril. The tape-wrapped sample was soaked in Freon TF for 3 minutes to fix and interconnect the Aflas component. This was followed by drying with the tape intact. The post-treated sample of this invention was coded Sample 3B-1. Scanning electron micrographs for Sample 2B and Sample 3B-1 are shown in Figures 6 to 9. Figures 6 and 7 show the lumen and outer surfaces of Sample 2B. Figures 8 and 9 show the lumen and outer surfaces of compliant Sample 3B-1. Both lumen and outer surface of said samples did not show any significant difference from each other.

Similar samples from Batch 2B were post-treated very similarly to the above procedure except for the soaking in Freon TF for 10 minutes (Sample 3B-2) and for overnight (Sample 3B-3).

EXAMPLE 4

Sample 2B was loaded onto a 6 mm mandril and tied at both ends. The entire sample was covered with a 6 mm latex balloon. The sample was soaked in Freon TF for 10 minutes and dried thereafter. The balloon was taken off and the post-treated sample of this invention was coded as 3B-4.

EXAMPLE 5

Compliance is measured by the following procedure.

Compliance Test

Equipment:

a) Laser gauge: Laser Mike® Model # 183 by Techmet
b) Graft proof tester

1. Cut test sample about 3″ (7.6 cm) in length.
2. Place a balloon inside the sample, and leave about 1/8″ (0.3 cm) longer than sample to be tested.

3. Place the sample over the brass connector barbs, and stay strap in place.
4. Take O.D. measurement before starting test. Invert the graft-loading fixture over the laser gauge, so the laser beam passes through the graft sample. Record the measurement on the laser gauge monitor.
5. Prime the sample with waterflow and zero monitor. Use the regulator attached to the reservoir to control the pressure. Make sure sample does not leak.
6. Turn the pressure up to 1.5 psi (80 mmHg), take a reading, increase pressure to 2.3 psi (120 mmHg), and take another reading. Continue reducing pressure to 1.5 psi and increasing to 2.3 psi. Take a reading at each point to get a total of 3 sets of readings.

| Example: | 1.5 psi | 2.3 psi |
|---|---|---|
| | 8.9874 | 9.2204 |
| | 8.9885 | 9.2107 |
| | 8.9986 | 9.2233 |

The compliance number for the samples are:

Group 1. Not radially enlarged.
Sample 1A Compliance = 0.03 $\times 10^{-2}$%/mmHg
Sample 1B Compliance = 0.33
Sample 1C Compliance = 0.54
Sample 1D Compliance = 0.70 (Impra I Control)
Sample 1E Compliance = 0.35 (Impra II Control)

Group 2. Radially enlarged.
Sample 2B Compliance = 0.40 $\times 10^{-2}$%/mmHg
Sample 2C Compliance = 0.41
Sample 2D Compliance = 0.66

Group 3. Radially enlarged and tape restrained.
Sample 3B-1 Compliance = 1.63 $\times 10^{-2}$%/mmHg
Sample 3B-2 Compliance = 1.59
Sample 3B-3 Compliance = 1.34

Group 4. Radially enlarged and ballon restrained.
Sample 3B-4 Compliance = 1.29 $\times 10^{-2}$%/mmHg

EXAMPLE 6

The test protocol for suture retention strength is measured by the following procedure.

SUTURE RETENTION TEST

1. Equipment used for the testing:
   a. Chantillon digital dynamometer
   b. Ethicon 6.0 prolene suture with C-1 taper needle
   c. A pair of haemostats
2. Set scale to read in grams. Assemble hook attachment to end of dynamometer.
3. Run suture through sample about 2 mm from edge of sample at a 90° angle. Make a loop with suture and clamp open end with a pair of haemostats.
4. Place loop end with haemostats over hook attachment on dynamometer and zero readout.
5. Hold sample with left hand, and secure scale with the right hand. Pull test sample away from hook attachment at 5 in/minute (12.7 cm/minute). Continue to pull until suture has torn through sample.
6. Take a reading when suture breaks through the sample. This will be the suture retention strength of the sample.
7. If the thread breaks during the testing, repeat test. This will not be a valid reading.

The suture retention strengths for the compliant samples and non-compliant sample are:

| | Suture Retention (Compliant) | Suture Retention (Non-compliant Counterpart) |
|---|---|---|
| 3B-1 | 439 grams | 427 grams |
| 3B-2 | 300 | 236 |
| 3B-3 | 681 | 481 |
| 3B-4 | 551 | 531 |

All the non-compliant counterparts were taken from the same expanded poly(tetrafluoroethylene)-elastomer (Aflas) tubing as control.

EXAMPLE 7

TENSILE STRENGTH

Tensile strength of a material is a maximum tensile stress, expressed in force per unit cross sectional area of the specimen, which the specimen will withstand without breaking. For porous materials, the cross-sectional area of solid polymer within the polymeric matrix is not the cross-sectional area of that porous specimen, but is equivalent to the cross-sectional area of the porous specimen multiplied by the fraction of solid polymer within the cross-section. This fraction of solid polymer within the cross-section is equivalent to the ratio of the specific gravity of the porous specimen itself divided by the specific gravity of the solid polymeric material which makes up the porous matrix. Thus, for example:

$\sigma$ = Standard tensile strength in psi
$P_1$ = Density of solid polymer, g/cc
$P_2$ = Density of expanded polymer, g/cc

$$\sigma_m = \frac{(\sigma)\ (P_1)}{(P_2)} = \text{Matrix tensile Strength}$$

$\sigma$ = 2500 psi $P_1$ = 2.2 (for solid PTFE)
$P_2$ = 0.40 (for expanded PTFE)

$$\sigma_m = \frac{(2500 \times (2.2)}{(0.40)} = 13{,}750 \text{ psi}$$

Matrix tensile strengths of grafts comprising various blends of polytetrafluoroethylene Aflas elastomer are shown in the following table:

| Polytetra-fluoroethylene Wt. % | Aflas Elastomer Wt % | Matrix tensile Strength |
|---|---|---|
| 90% | 10% | 6880 |
| 70% | 30% | 4285 |

EXAMPLE 8

By following the procedures of Examples 1 to 3 except for the final compliant graft with a 30% retraction during drying (with the balloon restraint still intact) will yield a longitudinally elastic graft. The basic physical properties, measured by conventional procedures, as compared to its non-compliant counterpart, are:

| | Compliant Graft | Non-Compliant Counterpart |
|---|---|---|
| Compliance, $10^{-2}$%/mmHg | 2.0 | 0.5 |
| Water-entry pressure, psi | 5.6 | 4.0 |
| Suture retention, grams | 460 | 460 |
| Wall thickness | 0.56 | 0.75 |
| Burst strength, psi | 88 | 86 |
| Longitudinal elasticity | excellent | excellent |

It appears the physical properties for the compliant graft are superior to the non-compliant graft.

## Claims

1. A process for the production of a porous compliant shaped article from a poly(tetrafluoroethylene) polymer, which process comprises first expanding a shaped article comprising poly(tetrafluoroethylene) crossed-linked with at least one elastomer, heating the unsintered shaped article at a temperature and for a time sufficient to cross-link said at least one elastomer to poly(tetrafluoroethylene), sintering the shaped article and radially expanding the sintered article.

2. A process according to Claim 1, wherein the radial expansion is up to 100% of the original inside diameter of the shaped article.

3. A process according to Claim 1 or Claim 2, wherein expansion occurs at a temperature of from 35°C to 342°C.

4. A process according to any one of Claims 1 to 3, wherein the radial expansion occurs at a temperature ranging from room temperature to 250°F (121°C).

5. A process according to any one of the preceding Claims, wherein the sintering temperature ranges from 342°C to 390°C.

6. A process according to any one of the preceding Claims, wherein the elastomer is cross-linked at a temperature from 150°F (65°C) to 350°F (177°C).

7. A process according to Claim 6, wherein the elastomer is cross-linked for a period of from 5 to 60 minutes.

8. A process according to any one of the preceding Claims, wherein the unsintered shaped article is expanded by linear stretching prior to sintering and radially expanding the shaped article.

9. A process according to any one of the preceding Claims, wherein the sintered shaped article is retracted to from 40% to 90% of its original length.

10. A process according to any one of the preceding Claims, wherein the elastomer is polyvinylidene fluoride-co-hexafluoropropylene, poly[tetrafluoroethylene-co-perfluoro(methylvinylether)], poly(tetrafluoroethylene-co-propylene), poly(vinylidene-co-chlorotrifluoroethylene), a silicone, a fluorosilicone, a fluoroalkoxy phosphazene, a segmented copolyester ether, a styrene butadiene block copolymer, a polyether, an acrylonitrile butadiene polymer, an isoprene polymer or a mixture of any two or more thereof.

11. A process according to any one of the preceding Claims, wherein the elastomer is present in an amount of from 2% to 50% by weight of the shaped article.

12. A process according to any one of the preceding Claims, further comprising:
compacting the radially expanded sintered article to its pre-radially expanded shape,
contacting said compacted sintered article with a solvent to partially dissolve said elastomer,
removing said solvent, and
ceasing the compaction of said sintered article to provide a porous longitudinally elastic compliant shaped article.

13. A process according to Claim 12, wherein said radially expanded sintered article is compacted by

fitting said article around a mandril having a smaller diameter than said article and applying means to hold said article on said mandril.

14. A process according to Claim 13, wherein said means to hold said article on said mandril is pressurized air.

15. A process according to Claim 13, wherein said means to hold said article on the mandril is porous tape or latex.

16. A process according to Claim 15, wherein said means is woven porous tape applied on said article at a wrapping angle of 70° with respect to the axis of the mandril.

17. A process according to any one of Claims 12 to 16, wherein the solvent is Freon TF, tetrahydrofuran or 1,1,1-trichloroethane.

18. A process according to any one of Claims 12 to 17, wherein said solvent is removed by evaporation.

19. A porous, longitudinally elastic vascular graft that is capable of dynamic local diameter or luminal volume change in response to pulsatile blood pressure change comprising poly(tetrafluoroethylene) cross-linked with an elastomer.

20. A graft according to Claim 19, wherein the elastomer is polyvinylidene fluoride-co-hexafluoropropylene, poly[tetrafluoroethylene-co-perfluoro(methylvinylether)], poly(tetrafluoroethylene-co-propylene), poly(vinylidene-co-chlorotrifluoroethylene), a silicone, a fluorosilicone, a fluoroalkoxy phosphazene, a segmented copolyester ether, a styrene butadiene block copolymer, a polyether, an acrylonitrile butadiene polymer, an isoprene polymer or a mixture of any two or more thereof.

21. A graft according to Claim 19, wherein the elastomer is present in an amount of from 2% to 50% by weight of the shaped article.

22. A graft according to Claim 19, wherein the internal diameter is from 1 mm to 10 mm.

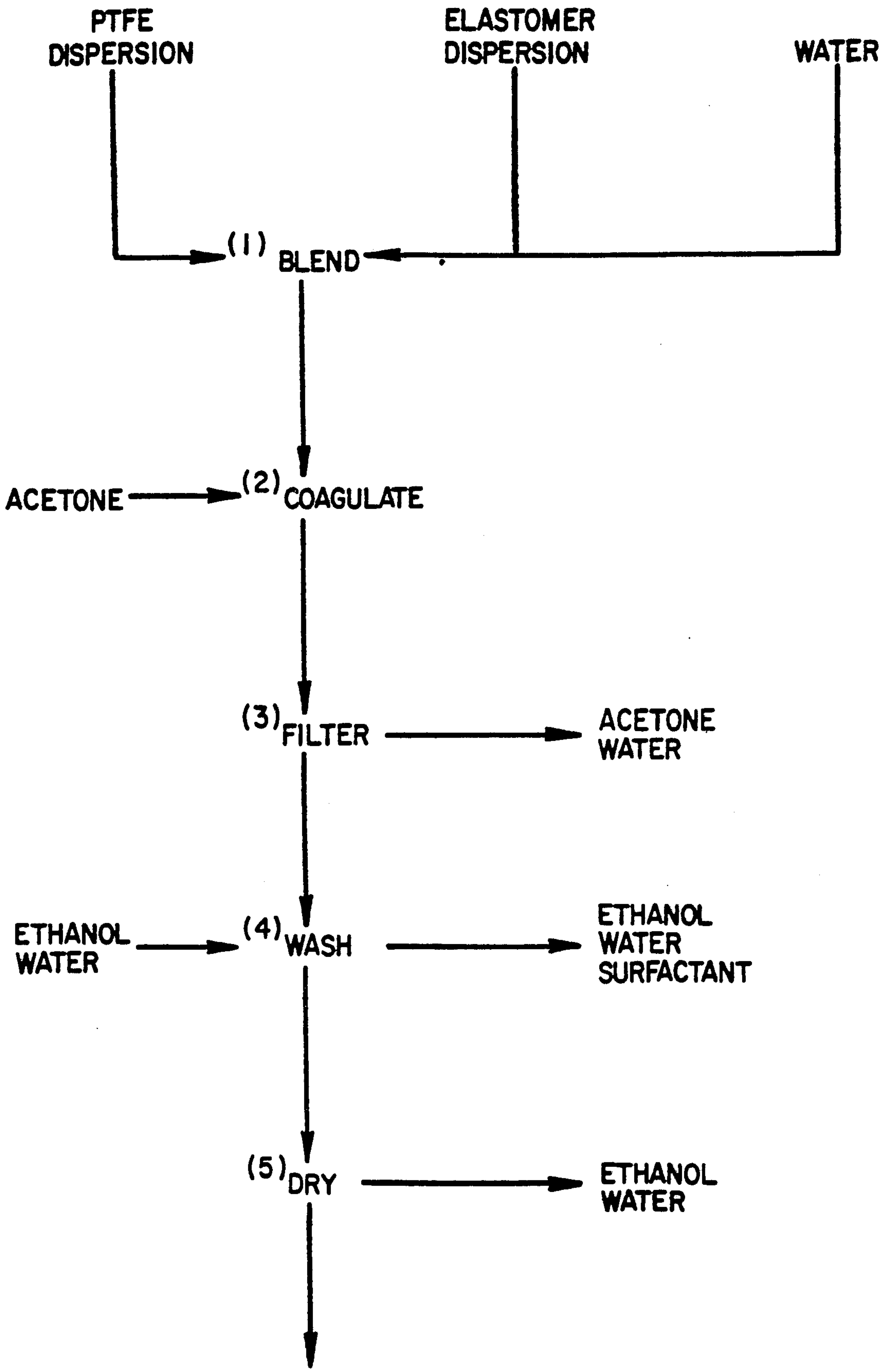
PTFE
DISPERSION
ELASTOMER
DISPERSION
WATER
(1) BLEND
ACETONE
(2) COAGULATE
(3) FILTER
ACETONE
WATER
ETHANOL
WATER
(4) WASH
ETHANOL
WATER
SURFACTANT
(5) DRY
ETHANOL
WATER

FIG. 1

0269449

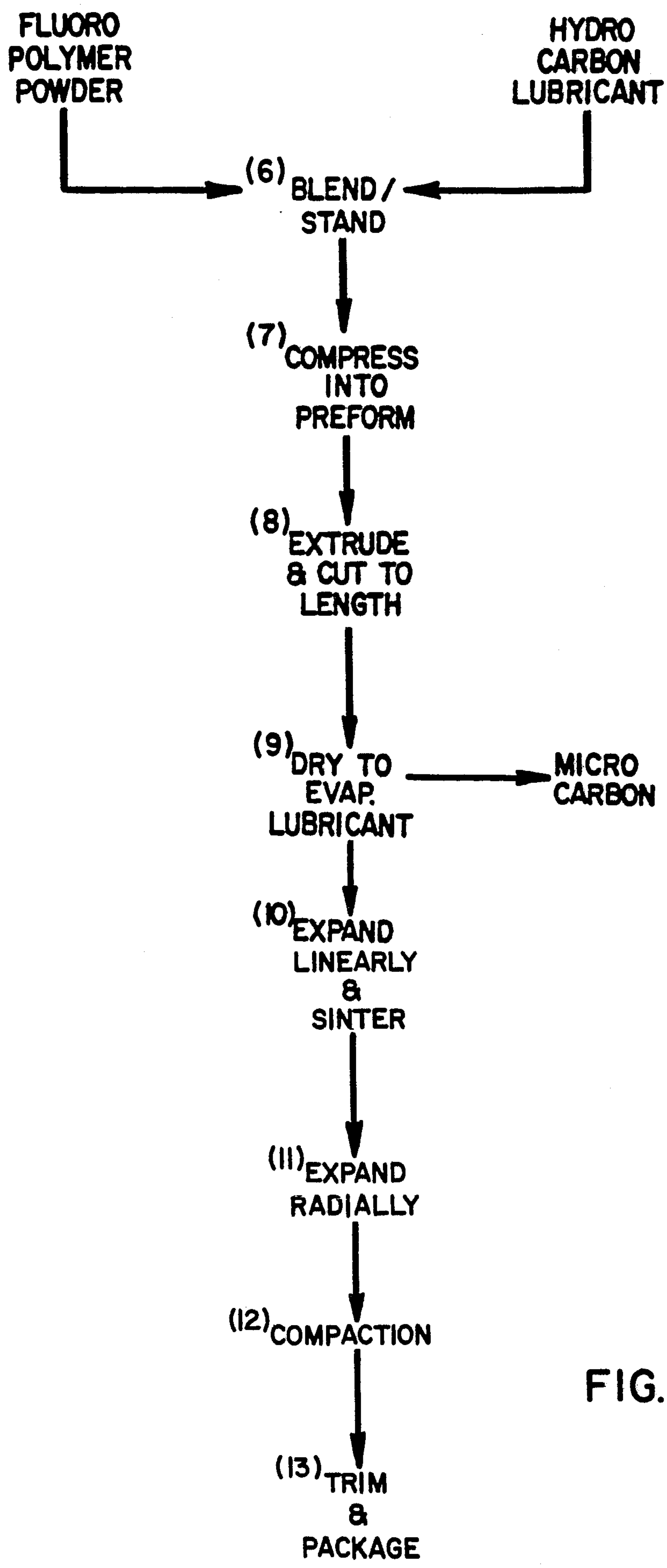


FIG. 2

0269449

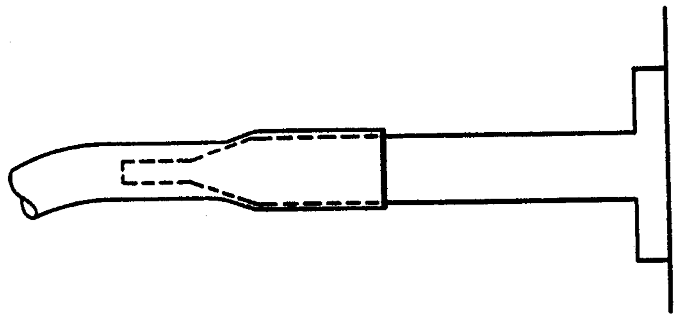

FIG. 5

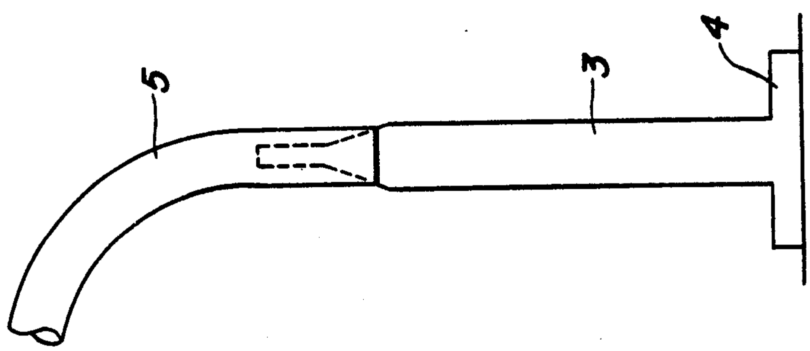


FIG. 4

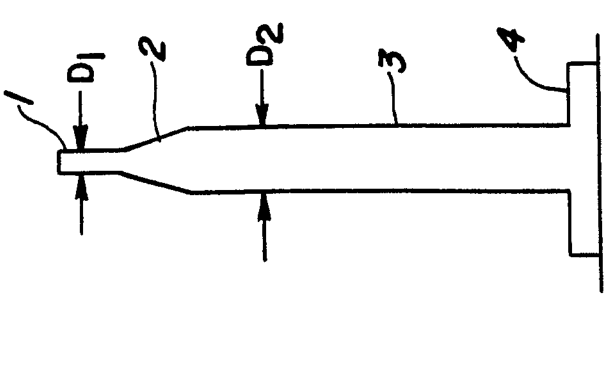


FIG. 3

05·02·88



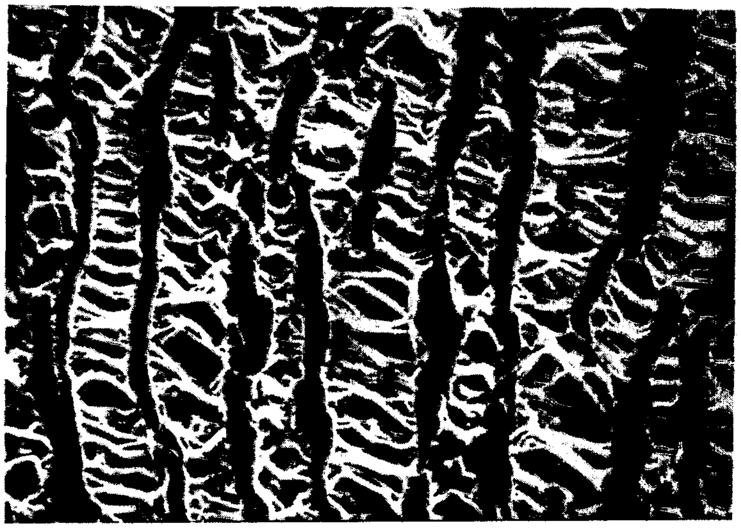

FIG. 6

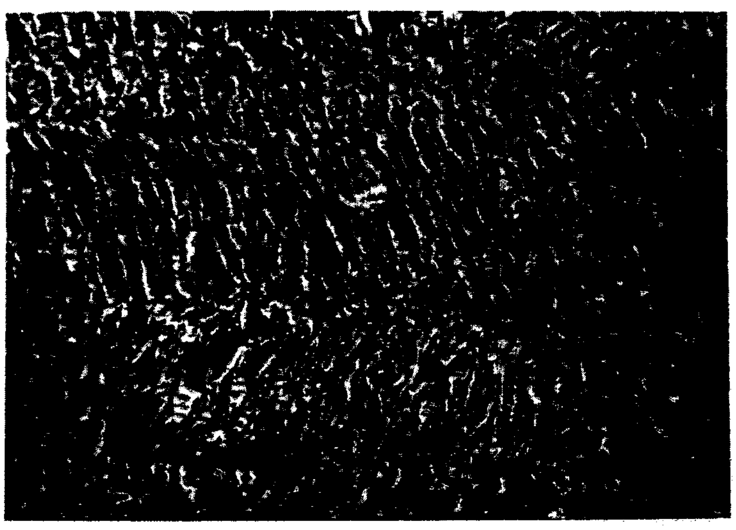

FIG. 7

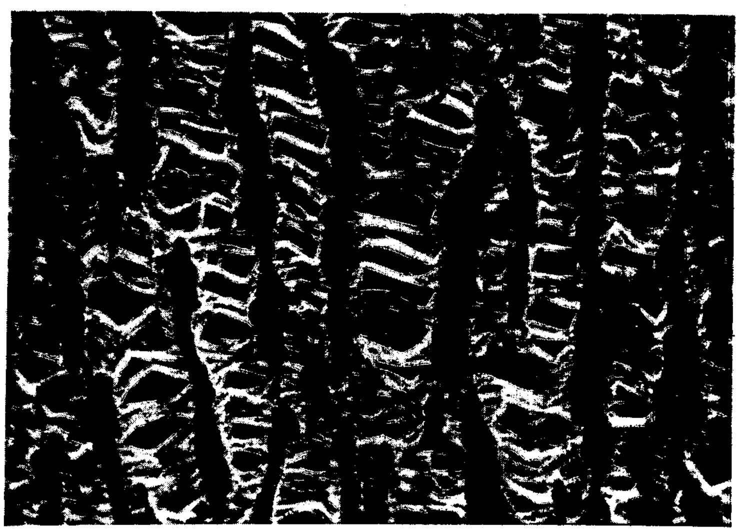

FIG. 8

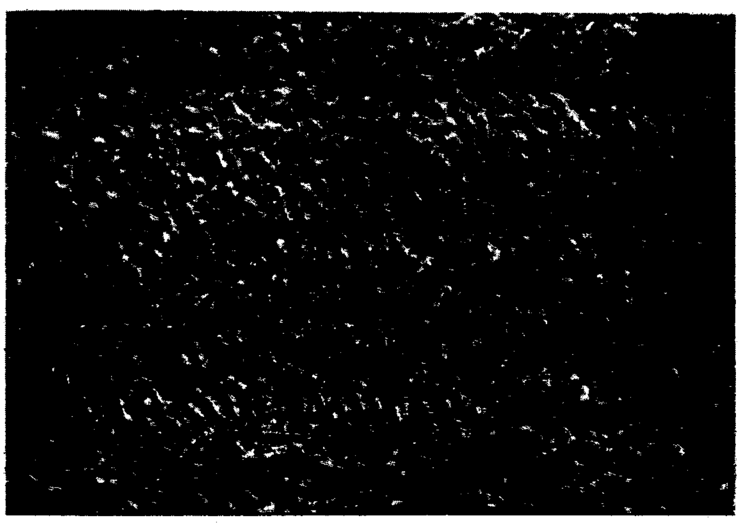

FIG. 9